(19) Europäisches Patentamt
European Patent Office
Office européen des brevets

(11) **EP 1 870 709 A1**

(12) **EUROPEAN PATENT APPLICATION**
published in accordance with Art. 153(4) EPC

(43) Date of publication:
**26.12.2007 Bulletin 2007/52**

(51) Int Cl.:
*G01N 33/53* (2006.01)   *C12M 1/00* (2006.01)
*C12N 15/09* (2006.01)   *G01N 37/00* (2006.01)

(21) Application number: **06730082.2**

(22) Date of filing: **27.03.2006**

(86) International application number:
**PCT/JP2006/306134**

(87) International publication number:
**WO 2006/101229 (28.09.2006 Gazette 2006/39)**

(84) Designated Contracting States:
**DE FR GB**

(30) Priority: **25.03.2005 JP 2005089403**
**28.10.2005 JP 2005315262**
**31.01.2006 JP 2006301565**
**02.03.2006 US 778100 P**

(71) Applicant: **NGK INSULATORS, LTD.**
**Nagoya-city, Aichi 467-8530 (JP)**

(72) Inventors:
• **YAMADA, Kazunari**
**u, Nagoya-city, Aichi, 4678530 (JP)**

• **YOSHIDA, Yasuko**
**u, Nagoya-city, Aichi, 4678530 (JP)**
• **ORIBE, Akinobu**
**u, Nagoya-city, Aichi, 4678530 (JP)**
• **TAKASE, Tomokazu**
**u, Nagoya-city, Aichi, 4678530 (JP)**

(74) Representative: **Paget, Hugh Charles Edward et al**
**Mewburn Ellis LLP**
**York House**
**23 Kingsway**
**London WC2B 6HP (GB)**

(54) **PROBE ARRAY AND PROCESS FOR PRODUCING THE SAME**

(57)     An object of the present invention is to provide a probe array having partitioned array regions with uniform surface chemical properties. The probe array of the present invention includes a substrate having a plurality of partitioned array regions where many probes are immobilized and a separator attached to the substrate and including partitions partitioning the array regions. The above object can be achieved by attaching the separator including the partitions capable of partitioning instead of forming hydrophobic regions on a surface of the substrate by printing or chemical treatment.

FIG.1

**Description**

Technical Field

The present invention relates to arrays of retained probes, or probe arrays, and methods for producing probe arrays, and specifically to, for example a probe array having a plurality of array regions, a separator designed therefor, a method for producing a probe array, and a method for hybridizing nucleic acid.

Background Art

In the field of general DNA microarrays with glass substrates, simultaneous examination of many samples has been attempted by forming a plurality of array regions on a single substrate (each array region is a probe-immobilized region where many probes are immobilized). Hybridization is performed with a glass cover placed on a glass substrate having a plurality of array regions. This method, however, can cause the problem that reaction solutions (solutions containing targets) in the adjacent array regions are contaminated with each other. Japanese Unexamined Patent Application Publication No. 2002-65274, for example, discloses that the reaction solutions are retained within the respective array regions by imparting hydrophobicity to partitions partitioning the array regions while maintaining hydrophilicity in the array regions, thereby avoiding contamination.

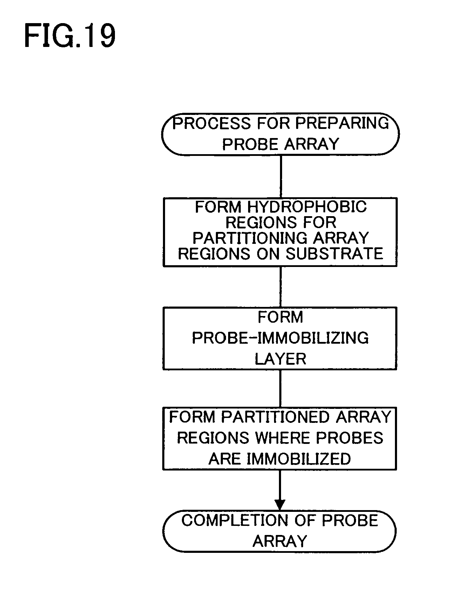

Referring to Fig. 19, an array, such as a DNA microarray, having many probes retained on a substrate is generally prepared by applying a surface finish for immobilizing the probes on a surface of the substrate, supplying the probes onto the surface of the substrate using any method such as inkjetting, and applying an immobilization treatment for immobilizing the probes on the surface of the substrate. Hydrophobic regions for partitioning the array regions are formed by printing or chemical treatment. These hydrophobic regions are fixed by heating the substrate to a temperature as high as several hundred degrees or immersing it in a particular liquid. Such treatments can degrade biological substances used as probes, such as nucleic acids or proteins, and finished surfaces. Conventionally, therefore, the step of forming the hydrophobic regions is performed prior to surface finishing and spotting, as shown in Fig. 19. Regions other than the hydrophobic regions formed on the substrate in advance are then subjected to surface finishing and spotting.

Disclosure of Invention

A study by the inventors, however, has found that the uniformity of hybridization reaction in the array regions tends to be decreased in comparison with the conventional art if the hydrophobic regions are formed on the substrate to partition the array regions in advance before the substrate undergoes surface finishing, probe spotting, and immobilization. Also, the efficiency of hybridization reaction in the array regions tends to be decreased if the hydrophobic regions are formed by, for example, printing or coating to partition the array regions. A further study has found that such problems in hybridization can result from nonuniform surface chemical properties of the array regions.

In addition, contamination of solutions under examination between array regions is a serious problem for arrays having a plurality of array regions. For example, contamination can occur when the arrays are transferred after the solutions under examination are applied to the array regions, for example, when the arrays are placed in a predetermined reaction chamber or when the arrays are set to, for example, an incubator under predetermined conditions.

For array having a plurality of array regions, furthermore, the individual array regions or a particular array is difficult to identify.

Accordingly, an object of the present invention is to provide a probe array having partitioned array regions with uniform surface chemical properties. Another object of the present invention is to provide a probe array having partitioned array regions that allow excellent reaction, such as hybridization, to occur between probes and solutions under examination. Another object of the present invention is to provide a probe array capable of suppressing or avoiding contamination between array regions. Another object of the present invention is to provide a technique for preparing a probe array having such array regions as described above in any regions on a substrate. Another object of the present invention is to provide a probe array having array regions that can readily be discriminated.

As a result of studies on the above problems, the inventors have found that a treatment for forming hydrophobic regions for partitioning array regions on a substrate prior to surface finishing, or the presence of such hydrophobic regions, impairs uniform surface chemical properties of the array regions. This results in nonuniform surface finishing, nonuniform shapes and sizes of probe spots, and nonuniform amounts of probe immobilized, thus contributing to increased variations in interaction reaction such as hybridization. In particular, the inventors have also found that such nonuniformities occur in peripheral portions of the array regions adjacent to the hydrophobic regions. The inventors have completed the present invention based on the founding that such conventional problems can be solved by eliminating a step of forming hydrophobic regions on a surface of a substrate by printing or chemical treatment and attaching a separator including partitions capable of partitioning array regions to, for example, a surface-finished substrate. Accordingly, the present invention provides the following means.

The present invention provides a probe array including: a substrate having one or more partitioned array regions where many probes are immobilized; and a separator attached to the substrate and including partitions partitioning the array regions. In the probe array of the invention, the probes are preferably nucleic acid probes. The probes are preferable protein probes. The substrate preferably has a plurality of the array regions.

In the probe array of the invention, the separator may be attached to a surface of a probe-immobilizing layer on the substrate, the probes being immobilized on the probe-immobilizing layer. In this case, the separator is preferably attached to the surface of the probe-immobilizing layer after the immobilization of the probes.

In the probe array of the invention, preferably the separator has a sheet shape and is fixed to the substrate with an adhesive layer disposed therebetween. The separator is preferably detachably fixed to the substrate.

In the probe array of the invention, hydrophobic regions may be disposed on at least part of the separator. In this case, the hydrophobic regions are preferably disposed on top surfaces of the partitions of the separator. The hydrophobic regions preferably have a water contact angle of 60° or more, more preferably 70° or more. The hydrophobic regions may contain a material selected from the group consisting of polycarbonates, polyolefins, polyamides, polyimides, acrylic resins, and fluorides and halides thereof.

In the probe array of the invention, regions surrounded by the partitions of the separator partitioning the array regions may constitute open cavities for interaction between the probes and samples under examination in the array regions.

In the probe array of the invention, the design area of the array regions defined by the partitions may be 0.3 to 2,000 mm$^2$, preferably 3 to 90 mm$^2$. The substrate may have 1 to 400 array regions, preferably 1 to 144 array regions.

In the probe array of the invention, the design area (Sd) of the array regions defined by the partitions is 90 mm$^2$ or less, and the percentage of effective probing area represented by the following equation (1) may be 70% or more:

$$\text{Percentage (\%) of effective probing area} = \text{Se(mm}^2)/\text{Sd(mm}^2) \times 100 \ \dots \ (1)$$

wherein Se is the area of a region where the coefficient of variation of signal intensity based on probe-sample interaction is 20% or less.

Probing means, for example, searching for, detecting, or examining an object based on interaction between the object and a probe capable of selective or specific interaction with the object. The coefficient of variation of signal intensity is the coefficient of variation of signal intensity based on interaction caused by supplying the object to interact with the probes on the probe array (standard deviation of signal intensity detected in minute regions of array regions/average of signal intensity detected in minute regions of array regions). The coefficient of variation of signal intensity can be determined by forming an appropriate number of minute regions (for example, spots), each containing probes of a single type, in each array region; supplying the object capable of interaction with the probes to perform hybridization; measuring a signal detected in each minute region; and determining the standard deviation and average of the measured signal intensities. The region where the coefficient of variation of signal intensity is 20% or less means a region including minute regions whose coefficient of variation of signal intensity is 20% or less among the appropriate number of minute regions in each array region. The outline of the region where the coefficient of variation of signal intensity is 20% or less can be defined by the outermost minute regions of a group of minute regions that satisfies the above coefficient of variation. The coefficient of variation of signal intensity is preferably determined based on 3 or more minute regions, more preferably 20 or more minute regions.

In the probe array of the invention, the coefficient of variation of signal intensity measured by probing is preferably 20% or less in regions other than those within the distance of 0.8 mm from the partitions in the partitioned array regions. The partitioned array regions preferably have a depth (d) of 10 to 240 μm.

In the probe array of the invention, the ratio (R) of the partitioned array regions which is represented by the following equation (2) may be 0.02 or less:

$$R = d(\text{mm})/\text{Sd(mm}^2) \ \dots \ (2)$$

wherein d is the depth of the array regions and Sd is the design area of the array regions.

In the probe array of the invention, the separator may be configured so that the partitions can have different heights. In the prove array, the separator may be configured so that the partitions can be reduced by removing at least part of the partitions. The separator may be configured so that the partitions can have different heights within the range of 10 to 1,000 $\mu$m. The partitions of the separator may have a height of 60 $\mu$m or more when a reaction is caused by supplying samples under examination to the probes and have a height of less than 60 $\mu$m when a signal from a reaction product is detected after the reaction. The area of the array regions may be 0.3 mm$^2$ or more. The separator may have a laminated structure. The separator may include a vulnerable portion where the partitions can be at least partially removed. The separator may be a laminate, and the vulnerable portion can be an interface between any layers of the laminate.

In the probe array of the invention, the separator may include overhanging portions protruding from at least part of the tops of the partitions to above the array regions. The overhanging portions may be disposed around the entire peripheries of the array regions. The overhanging portions may include segments protruding from the partitions to above the array regions with hydrophobic regions disposed in at least regions of the segments where the segments can come in contact with solutions under examination.

In the probe array of the invention, the height of lowermost ends of the overhanging portions (height from the surface of the substrate) may be 40 to 990 $\mu$m. The overhanging portions may protrude within such a distance as not to reach the probes immobilized in the array regions. Regions defined by inner peripheral surfaces of the overhanging portions may be regions where the probes are immobilized in the array regions. At least part of overhanging portions may be integrated with and/or removed from the rest of the partitions or the entire overhanging portions may be integrated with and/or removed from the rest of the partitions.

In the probe array of the invention, the separator may constitute at least part of the height of the partitions partitioning the array regions. The substrate may constitute at least part of the height of the partitions partitioning the array regions.

The probe array of the invention may further include a discrimination layer having a color and/or image that allows discrimination of the array regions. In this case, the discrimination layer may be disposed on the substrate side of the separator. Preferably, the separator includes the discrimination layer. The discrimination layer may be formed by printing. The discrimination layer may have a color that allows discrimination of the array regions, or may have one or more marks selected from letters, numbers, symbols, and graphics to allow identification of the array regions. The discrimination layer may have both a color and an image.

The present invention also provides a separator including partitions for partitioning one or more array regions where many probes are immobilized on a substrate. In the separator of the invention, hydrophobic regions are preferably disposed on top surfaces of the partitions of the separator. The substrate may have a plurality of the array regions. The separator may be configured so that the partitions can have different heights. The separator may further include overhanging portions protruding from at least part of the tops of the partitions to above the array regions.

The separator of the invention may further include a discrimination layer having a color and/or image that allows discrimination of the array regions. In this case, the discrimination layer is preferably disposed on the substrate side. The discrimination layer may be formed by printing.

The separator of the invention may have a laminated structure. The separator is preferably used for the probe array according to any of the above probe arrays.

The present invention also provides a probe array having one or more array regions where many probes are immobilized. The array regions are partitioned by the above separator. The probe array of this embodiment is a probe array having unpartitioned array regions. The above separator is attached to the probe array of this embodiment to provide a probe array having partitioned array regions.

The present invention also provides a method for producing a probe array. The method includes: a step of immobilizing many probes in one or more array regions on a substrate; and a step of partitioning the many probes into the array regions by attaching a separator to a surface of the substrate where the probes are immobilized, the separator including partitions capable of partitioning the surface of the substrate. In the method of the invention, a plurality of the array regions may be formed.

The present invention also provides a method for hybridizing nucleic acid. The method includes a step of supplying solutions containing nucleic acid samples under examination into the array regions of the probe array of a nucleic acid probe to hybridize the samples under examination with the probes. In the method, the probe array has open cavities for hybridization in the array regions, and the hybridization step may be performed under humidified conditions by supplying the solutions under examination into the open cavities.

The present invention also provides a reaction method using a probe array is provided. The method includes a step of supplying solutions under examination into the array regions of any of the above probe arrays with protein probes provided thereon to cause a reaction with the probes.

The present invention provides a reaction method using a probe array. The method includes: a reaction step of causing a reaction by supplying solutions under examination into one or more array regions, partitioned by partitions having a first height, where many probes are immobilized on a substrate of the probe array; and a subsequent step of cleaning or detection of a reaction product after the reaction step, the subsequent step being performed without the partitions present or with the height of the partitions reduced to a second height lower than the first height.

The reaction method of the invention may further include a step, prior to the reaction step, of forming the partitions having the first height on the substrate after the probes are immobilized on the substrate. In the reaction method of the invention, the partitions having the first height in the reaction step may include overhanging portions protruding from at least part of the tops of the partitions to above the array regions; and the height of the partitions may be reduced to the second height by removing the overhanging portions in the subsequent step. The partitions may be formed by attaching a separator capable of partitioning the array regions to a surface of the substrate. The separator may be a laminate.

Brief Description of the Drawings

Fig. 1 shows diagrams of an example of a probe array.

Fig. 2 is a detailed diagram of the probe array.

Fig. 3 is a diagram showing an example of a partition pattern of a separator sheet.

Fig. 4 is a diagram showing another example of the partition pattern of the separator sheet.

Fig. 5 is a diagram illustrating the concept of the percentage of effective probing area.

Fig. 6 is a flow chart of an example of a process for preparing a probe array.

Fig. 7 is a diagram showing probe array regions (unpartitioned) formed on a substrate in the process for preparing a probe array.

Fig. 8 is a diagram showing a pattern of array regions of a nucleic acid probe array of an example.

Fig. 9 is a diagram showing the structure of a separator sheet of the example.

Fig. 10 is a graph showing the relationship between the distance from partitions and the CV of fluorescence intensity.

Fig. 11 is a graph showing a difference in percentage of effective hybridization area between the example and a comparative example.

Fig. 12 is a graph showing the relationship between the depth of the array regions and fluorescence intensity.

Fig. 13 is a graph showing the relationship between water repellency and fluorescence intensity.

Fig. 14 is a graph showing the relationship between the ratio R of the depth of the array regions to the design area of the array regions and fluorescence intensity (at a depth of the array regions of 0.25 mm).

Fig. 15 is a graph showing the relationship between the ratio R of the depth of the array regions to the design area of the array regions and fluorescence intensity (at a depth of the array regions of 0.1 mm).

Fig. 16 is a diagram showing the laminated structure of a separator sheet prepared in Example 5.

Fig. 17 is a diagram showing the laminated structure of another separator sheet prepared in Example 5.

Fig. 18 shows a diagram showing the laminated structure of an array prepared in Example 6 and a plan view thereof.

Fig. 19 is a flow chart of an example of a conventional process for preparing a probe array.

Best Mode for Carrying Out the Invention

A probe array of the present invention includes a substrate having partitioned array regions where many probes are immobilized and a separator including partitions partitioning the array regions from the surroundings thereof. In the probe array of the present invention, the separator having the partitions partitioning the array regions from the surroundings thereof is attached to the substrate. That is, the array regions are partitioned without forming hydrophobic regions on the substrate by, for example, printing with a water-repellent material or immersing. The probe array of the present invention can therefore eliminate nonuniformities in a variety of operations, including nonuniform surface chemical properties of the substrate, nonuniform surface finishing, and nonuniform probe shapes, probe sizes, and amounts of probe immobilized, due to the above treatments performed before surface finishing or the presence of hydrophobic or water-repellent regions on the substrate in the probe array preparation stage. As a result, the partitioned array regions can be formed with uniform surface chemical properties inside the array regions. In addition, the partitioned array regions allow excellent probing with, for example, a uniform pattern and amount of probe immobilized. As a result, even probing for many samples and items can be efficiently performed.

A separator of the present invention includes partitions for partitioning one or more array regions where many probes are immobilized on a substrate. This separator can be attached to a probe array having one or more array regions where many probes are immobilized, thus providing a probe array of the present invention having partitioned array regions. This probe array can provide the same advantages as described above.

A method of the present invention for producing a probe array includes a step of immobilizing many probes in a pattern where one or more array regions can be formed on a substrate and a step of partitioning the many probes into the array regions of the pattern by attaching a separator to a surface of the substrate where the probes are immobilized.

The separator includes partitions capable of partitioning the surface of the substrate into one or more regions. According to this method, many probes are immobilized in a pattern corresponding to the array regions to be partitioned before the separator is used to partition the many probes into the array regions. Hence, the array regions are partitioned without forming hydrophobic regions on the substrate by, for example, printing with a water-repellent material or immersing. This eliminates the need for a treatment for forming hydrophobic regions and the nonuniformities due to the presence of hydrophobic regions, so that a preferred probe array can be produced. Preferably, a plurality of array regions is formed.

A method for hybridizing nucleic acid includes a step of supplying solutions containing samples under examination into the array regions of the probe array to hybridize the samples under examination with the probes. This method allows for excellent hybridization in the array regions of the probe array because the method does not involve various problems encountered by conventional partitioning methods.

In the probe array of the present invention, the partitions of the separator can be defined to different heights. This allows for excellent reaction between the probes and the solutions under examination. Specifically, the array regions can be partitioned by the heights required in, for example, production or reaction steps of the probe array, including probe immobilization, blocking, reaction, cleaning, and signal detection. In probe immobilization and surface finishing, for example, the partition height can be defined to such a height that the partitions do not obstruct these treatments, or to zero (no partitions present). In the reaction, for example, the partition height can be defined to such a height that the partitions can avoid or suppress contamination of the solutions under examination. In cleaning and signal detection, the partition height can be defined to a lower height or to zero. The partition height can be reduced to zero if the separator is configured so as to be detachable from the substrate. In addition, the adjustment of the partition height can increase the flexibility of the amount of solution supplied to cavities including the array regions. This allows the reaction to occur readily with an appropriate amount of solution under examination based on the type of solution and the type of reaction. After the reaction, the partition height may be maintained or increased if, for example, a coloring reaction is induced for each array region. In other cases, the partitions may be removed or lowered.

The probe array of the present invention can also include overhanging portions protruding from at least part of the tops of the partitions to above the array regions. When the solutions under examination are supplied into the cavities including the array regions partitioned by the partitions, the overhanging portions can effectively avoid or suppress contamination of the solutions under examination between the array regions. This allows for excellent reaction between the probes and the solutions under examination. If the overhanging portions have hydrophobic regions in sites to be in contact with the solutions under examination, the overhanging portions can more effectively avoid or suppress contamination of hydrophilic solutions under examination. In addition, the overhanging portions can provide a sufficient effect of suppressing contamination with the partitions 14 lowered. The partitions having the overhanging portions can be configured so that at least part of the overhanging portions can be integrated with or removed from the rest of the partitions. In this case, the overhanging portions can deliver the effect of suppressing contamination without obstructing production of the probe array or a series of operations and treatments.

A reaction method using the probe array of the present invention includes a step of allowing the probes to react with solutions under examination with the array regions partitioned by partitions of a first height, and subsequent steps following the reaction step, such as cleaning and detection, are performed with the array regions partitioned by a second height lower than the first height or without the partitions present. This method allows for excellent reaction between the probes and the solutions under examination. As described above, a preferred reaction can be achieved on the probe array by adjusting the partition height according to need, that is, using high partitions in the reaction and low partitions in the subsequent steps, because the desired properties (such as height) of the partitions partitioning the array regions depend on the array production or reaction steps.

The probe array, separator, method for producing a probe array, and method for hybridizing nucleic acid of the present invention will now be described.

(Probe array)

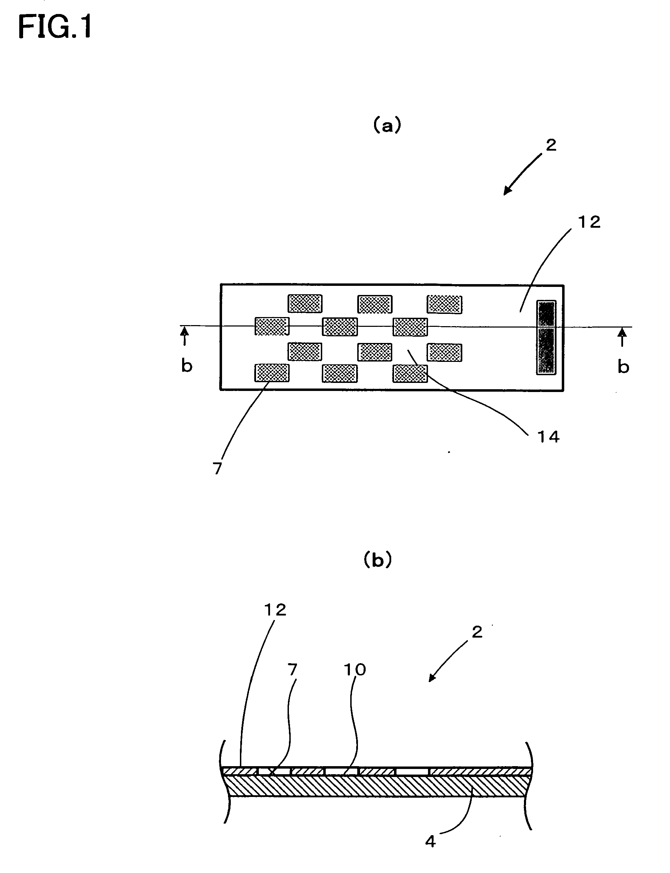

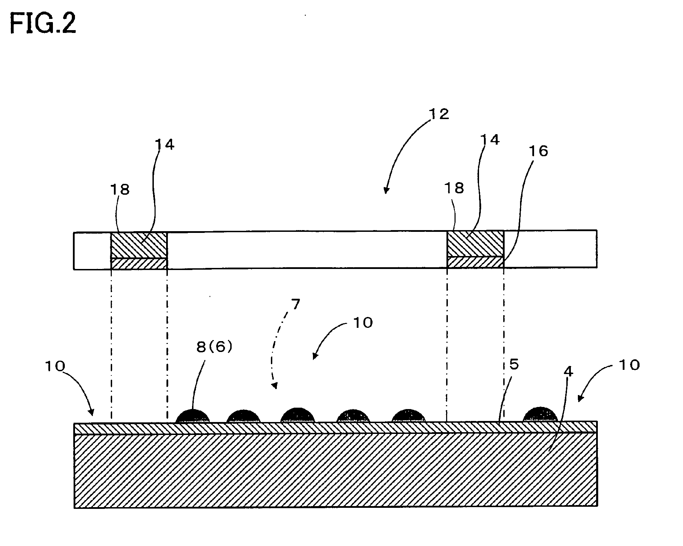

Fig. 1 shows an embodiment of a probe array 2 of the present invention, and Fig. 2 shows a detailed diagram thereof. The probe array 2 includes a substrate 4, the substrate 4, and a separator 12. Spot-like minute regions 8 where many probes 6 are immobilized are arranged on the substrate 4. These minute regions 8 are arranged so as to form, for example, each array region 10.

(Probe)

The probes 6 used in the present invention are not particularly limited and can be of any material, such as a nucleic acid, a protein, or a synthetic or natural compound, that can provide a detectable signal resulting from a direct or indirect interaction between the probes and samples contained in solutions under examination on the array regions 10. Various types of interactions are available, examples including interactions, called hybridization, based on base-paring of nucleic acids and specific bonds between proteins and nucleic acids, proteins and low-molecular-weight compounds, and between nucleic acids and low-molecular-weight compounds.

Most typically, a nucleic acid can be used as the probes of the probe array of the present invention. The nucleic acid used can be any nucleic acid that at least partially hybridizes with another nucleic acid through base-pairing. The term nucleic acid used herein is a concept that encompasses both natural and synthetic oligomers and polymers of nucleotides and also encompasses DNAs such as genomic DNAs and cDNAs, PCR products, RNAs such as mRNAs, and peptide nucleic acids. Nucleic acids can provide a detectable signal through an interaction based on a hybridization reaction due to base-pairing or a specific bond with a protein or a low-molecular-weight compound.

Examples of the protein used include enzymes, antibodies, antigens, and receptors, any of which can provide a detectable signal because they react specifically with substrates, antigens, antibodies, and nucleic acids, respectively. Examples of other synthetic or natural compounds include a variety of reagent candidate compounds synthesized by combinatorial chemistry. In the present invention, the probes 6 used are not limited to the above examples and can be of any material that allows detection of a target compound.

For example, the probes 6 are present on the substrate 4 as the minute regions 8, which are about 50 to 500 μm in diameter. In the present invention, the method and pattern used for immobilizing the probes 6 on the substrate 4 are not particularly limited, and examples thereof include all forms known at the time of the present application. Accordingly, examples of the method used include contact probe immobilization methods using pins, noncontact probe immobilization methods by inkjetting, and methods of synthesizing the probes 6 on the substrate 4.

(Substrate)

The shape and material of the substrate 4 are not particularly limited, and various materials can be used, including those used for conventional DNA chips and DNA microarrays. Examples include ceramics such as silicon-based ceramics, including glass, silicon dioxide, and silicon nitride; resins such as silicone, polymethylmethacrylate, and poly(meth)acrylate; and metals such as gold, silver, and copper. An appropriate coating can be used to impart desired surface properties. In particular, glass substrates, silicone, and acrylic resins can be used.

Referring to Fig. 2, the substrate 4 preferably has a probe-immobilizing layer 5 formed using a treatment appropriate to the material of the probes 6, such as a nucleic acid, to immobilize the probes 6 on the substrate 4. The type of the probe-immobilizing layer 5 is not particularly limited. For example, if a nucleic acid is used as the probes 6 to be immobilized, the probe-immobilizing layer 5 used can be, for example, a positively charged polymer layer, such as a polylysine layer, or a polymer layer having an aldehyde or carboxyl group that can bind with a functional group in the nucleic acid or a reactive group bound to the nucleic acid. Preferably, regions where the probes 6 are to be immobilized on the substrate 4 are substantially flat; this also encompasses minute three-dimensional shapes such as porous shapes.

(Array region)

The array regions 10 are disposed on the substrate 4. The array regions 10 are regions where the minute regions 8 are formed. The array regions 10 are formed as clusters of many minute regions 8. One or more array regions 10, preferably a plurality of array regions 10, are disposed on the substrate 4. Different groups of probes 6 may be immobilized in different array regions 10. In the probe array 2 of the present invention, the array regions 10 are at least partitioned from the surroundings thereof or from each other. The array regions 10 are preferably partitioned so that when the solutions under examination are supplied into the partitioned array regions 10, a predetermined amount of one of the solutions under examination is retained in each of the array regions 10 without flowing out to the surroundings thereof or into other adjacent ones of the array regions 10. Solutions containing many samples (or different types of solutions) under examination can be simultaneously assayed by supplying the solutions containing different samples under examination into the array regions 10.

The array regions 10 are preferably partitioned so as to define cavities 7. The cavities 7 can be, for example, open cavities, with at least part of the array regions 10 being open. Typically, the cavities 7 are recessed cavities that are open upwardly. The cavities 7 can also be closed cavities having openable/closable inlets for the solutions under examination. The cavities 7 are defined by the separator 12 described later. The cavities 7 are spaces where the probes 6 interact with the samples under examination. If the probe array 2 is a nucleic acid probe array, the cavities 7 serve as spaces for hybridization reaction.

The depth of the cavities 7 can be partially defined by recesses or protrusions of the substrate 4 itself. For example, the substrate 4 itself may have shallow wells corresponding to the array regions 10 or, conversely, may have partitions with a height corresponding to part of the height of partitions 14 partitioning the flat array regions 10.

The depth of the cavities 7 (synonymous with the height of the partitions 14 described later) is preferably 10 to 240 μm. Within this range, the solutions under examination can be retained in the cavities 7 with such a thickness as to ensure convection of the solutions under examination and diffusion of the samples under examination while suppressing the effect of the partitions defining the cavities 7, thus contributing to highly sensitive detection. The depth of the cavities 7 is more preferably 150 $\mu$m or less, still more preferably 100 $\mu$m or less. The depth of the array regions 10, as described later, can be defined as the height from the surface of the substrate 4 where the probes 6 are immobilized to the top surfaces of the partitions 14. This distance can readily be measured using, for example, various digital length-measuring machines. This cavity structure is preferred for probing in an aqueous medium, such as in the case of nucleic acid hybridization.

The relationship between the depth of the cavities 7 and the design area of the array regions 10 is preferably determined such that the ratio (R) represented by the following equation (2) is 0.02 or less:

$$R = d(mm)/Sd(mm^2) \ldots (2)$$

where d is the depth of the array regions and Sd is the design area of the array regions. If the ratio R falls within the above range, the cavities 7 can ensure convection of the solutions under examination and diffusion of the samples under examination while suppressing the effect of the partitions 14 defining the cavities 7, thus contributing to highly sensitive detection. This cavity structure is preferred for probing in an aqueous medium, such as in the case of nucleic acid hybridization.

The size of the array regions 10, that is, the design area of the array regions (hybridization area), is preferably 0.3 to 2,000 mm$^2$. Within this range, the coefficient of variation of signal intensity based on probe-sample interaction is well suppressed. If the design area of the array regions 10 is 0.3 mm$^2$ or less, the partitions 14 tend to obstruct the convection of the solutions under examination and the diffusion of the samples under examination. If the design area of the array regions 10 is 2,000 mm$^2$ or more, the coefficient of variation of signal intensity is difficult to suppress only by the convection of the solutions under examination and the diffusion of the samples under examination. More preferably, the design area of the array regions 10 is 3 to 90 mm$^2$. The design area of the array regions refers to the internal area of each array region which is defined by the partitions 14 of the separator 12 described later. The number of array regions 10 on the substrate 4 is not particularly limited, although the substrate 4 preferably has 1 to 400 array regions 10, more preferably 1 to 144 array regions 10. In terms of compatibility with existing infrastructures, 400 or less array regions 10 are preferred for sizes similar to those of 96-well plates, and 144 or less array regions 10 are preferred for sizes of glass slides.

(Separator)

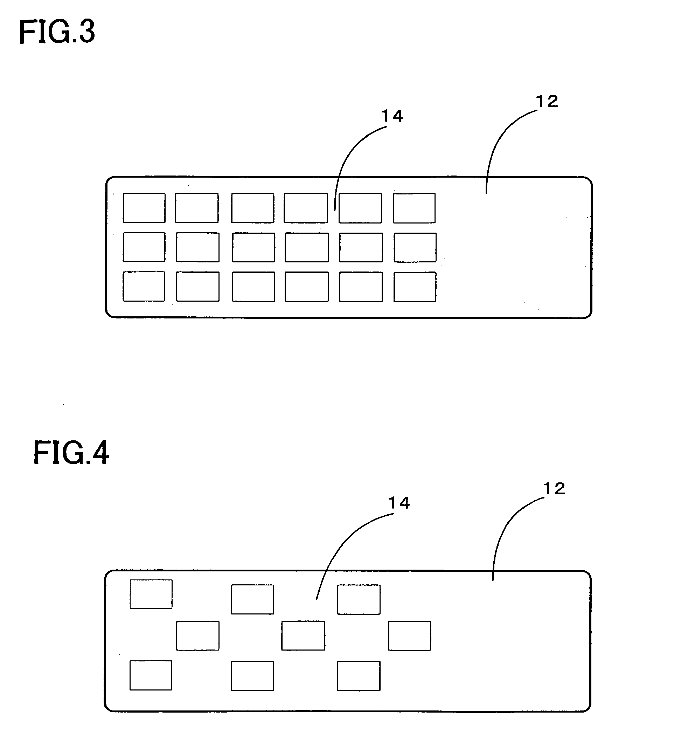

The separator 12 is disposed on the substrate 4 to partition the array regions 10. As shown in Figs. 1 to 4, the separator 12 includes the partitions 14 for partitioning the array regions 10. The partitions 14 of the separator 12 are used to partition the array regions 10 from the surroundings thereof and correspond to the size and shape of the array regions 10 to be partitioned. In the example shown in Fig. 3, the partitions 14 cross each other in a grid pattern so that the adjacent array regions 10 can be partitioned. In the example shown in Fig. 4, the partitions 14 are formed in a staggered pattern so that the array regions 10 can be partitioned in a staggered pattern. The pattern of the array regions 10 thus partitioned is not limited to the examples shown in Figs. 3 and 4, and one or more array regions 10 may be partitioned in any pattern. The partitions 14 of the separator 12 are preferably formed in such a pattern as to surround the entirety of each of the array regions 10.

While the pattern of the partitions 14 of the separator 12 varies with the size, number, and shape of the array regions 10 to be partitioned, the separator 12 itself preferably has a sheet shape because its flat surface can readily be attached to the substrate 4. The sheet shape means any shape that can be regarded as a sheet shape in its entirety in terms of the thickness and external shape (such as width and length) of the separator 12, even though the partitions 14 are formed in a skeleton pattern.

The separator 12 may be attached to the substrate 4 so that the array regions 10 can be partitioned. The separator 12 may be placed on the substrate 4 and held together using, for example, a jig. More preferably, as shown in Fig. 2, the separator 12 is fixed to the substrate 4 with an adhesive layer 16 disposed therebetween. The adhesive layer 16 allows the separator 12 to be readily attached to the substrate 4 after the immobilization of the probes 6. Specifically, the adhesive layer 16 used may be a strong adhesive capable of lasting adhesion or a tacking agent capable of easy separation. Bonding using the adhesive layer 16 is particularly preferred if the separator 12 has a sheet shape.

The separator 12 is preferably configured so that the height thereof, that is, the height of the partitions 14, can be changed according to need. To allow an increase or decrease in height, the separator 12 preferably has a laminated structure whose constituent layers can be integrated and/or removed. That is, different heights can be defined if the separator 12 has a laminated structure including two or more constituent layers and is configured so that any constituent layers can be removed from at least one interface. The separator 12 may also have a laminated structure whose constituent layers can be stacked on top of each other. In addition, the separator 12 may have a laminated structure that allows both removal and integration of the constituent layers.

Preferably, any interface of the laminated structure is a vulnerable portion, and the laminated structure is configured so that the constituent layers can be integrated and/or removed at the vulnerable portion. For example, the laminated structure may be configured so that the constituent layers can be integrated and/or removed using an adhesive or tacking layer or the adhesiveness or tackiness of the constituent layers themselves. Such a structure allows easy adjustment of the adhesion strength between the constituent layers, thus readily ensuring, for example, ease of handling for height adjustment. Alternatively, a mechanical fit, for example, may be used so that the constituent layers can be integrated and/or removed. According to need, the laminated structure can have one or more interfaces where the constituent layers can be integrated and/or removed.

The vulnerable portion may be positioned at a predetermined height in the laminated structure so that the height of the separator 12 can be changed. If any site of the separator 12 is vulnerable in terms of structure or material, the height of the separator 12 can be reduced by removing part of the separator 12 at that site, without employing a laminated structure.

The separator 12, which includes the partitions 14 whose height can be changed, is preferably configured so that the height of the separator 12 can be changed within the range of, for example, 10 to 1,000 $\mu$m. Within this range, the height of the partitions 14 can be defined so that the partitions 14 can avoid or suppress contamination of the solutions under examination in the reaction, and can also be defined so that the partitions 14 do not obstruct cleaning or signal detection after the reaction. More specifically, the height of the partitions 14 is preferably 60 $\mu$m or more in the reaction. If the height is 60 $\mu$m or more, the partitions 14 can well suppress contamination between the array regions during array transfer while maintaining sufficient amounts of solutions supplied. More preferably, the height of the partitions 14 is 200 $\mu$m or more. If the height is 200 $\mu$m or more, the reaction inside the cavities 7 including the array regions 10 is facilitated while the liquid levels of the solutions supplied into the cavities 7 do not exceed the height of the partitions 14. On the other hand, the height of the partitions 14 is preferably less than 60 $\mu$m in the subsequent steps such as cleaning and signal detection. If the height is less than 60 $\mu$m, a cleaning solution can spread smoothly over the array regions 10 in the cleaning, and the probe array 2 can be successfully applied to general existing apparatuses (infrastructures) for signal detection. More preferably, the height is 50 $\mu$m or less. The height of the partitions 14, however, is preferably 40 $\mu$m or more even in the subsequent steps. If the height is 40 $\mu$m or more, the partitions 14 can suppress contamination of the solutions under examination between the array regions 10 after the reaction when the height of the partitions 14 is reduced by removing the top of the partitions 14.

The separator 12 may also include the overhanging portions protruding from at least part of the tops of the partitions 14 to above the array regions 10. These overhanging portions may be formed around the entire partitions 14 surrounding the array regions 10 or only partially around the partitions 14. Examples of the overhanging portions formed partially around the partitions 14 include overhanging portions formed only on either pair of opposing partitions and many overhanging portions formed discontinuously at appropriate intervals around the entire partitions 14.

In addition, the shape of the overhanging portions is not particularly limited. The overhanging portions may protrude parallel to the surface of the substrate 4 or diagonally downward. In addition, the overhanging portions may be cut such that the inner peripheral surfaces thereof are tapered upward in a dome shape, for example, with respect to the surface of the substrate 4. The amount of overhang of the overhanging portions preferably falls within such a range that they do not reach the probes immobilized in the array regions 10. If the overhanging portions reach the probes, they can cause a problem, for example, that bubbles remain over the probes when the solutions containing the samples under examination are supplied. In other words, if the separator 12 has the overhanging portions, the regions where the probes are immobilized in the array regions 10 are defined by the outermost ends of the overhanging portions. Accordingly, the ends of the overhanging portions are preferably positioned within the distance of 0.8 mm from the partitions 14.

In addition, the lowermost ends of the overhanging portions are preferably positioned at a height of 40 to 990 $\mu$m. If the height is 40 $\mu$m or more, the overhanging portions can be removed by bending without contact with the array regions or damage thereto. More preferably, the height is 60 to 500 $\mu$m.

The overhanging portions preferably have hydrophobic regions 18 in sites where they can come in contact with the solutions under examination. The hydrophobic regions 18 can be disposed to more effectively avoid or suppress contamination of the solutions under examination. The sites where the overhanging portions can come in contact with the solutions under examination include, for example, the bottom surfaces of the overhanging portions opposite the surface of the substrate 4 and the inner peripheral surfaces thereof. Preferably, the hydrophobic regions 18 are also formed on the top surfaces of the overhanging portions if the overhanging portions are formed at a relatively low height, for example, with the uppermost ends thereof positioned at a height of less than 60 $\mu$m, and the top surfaces of the overhanging portions are exposed because, for example, they are formed on the tops of the partitions 14. To provide the hydrophobic regions 18 on the overhanging portions, the formation of the overhanging portions themselves using a hydrophobic material described later is typically preferred.

The overhanging portions may be disposed on the partitions 14 or the separator 12 in any manner. The overhanging portions may be disposed on the tops of the partitions 14 so that the overhanging portions can be attached or removed alone, or may also be disposed in advance as part of, for example, the tops of the partitions 14. Alternatively, the overhanging portions may be patch-shaped such that they partially cover the peripheries of the openings (defined by the top edges of the partitions 14) of the cavities 7 including the array regions 10 surrounded by the partitions 14.

The overhanging portions can be disposed to suppress contamination of the solutions under examination while reducing the height of the partitions 14.

(Discrimination layer)

The separator 12 can have a discrimination layer. The discrimination layer may have a color and/or image so that one or more (preferably two or more) array regions 10 can be discriminated. In particular, the discrimination layer preferably has a color, for example, so that the array regions 10 can be visually discriminated. The discrimination layer is formed around the array regions 10 so that they can be discriminated. Accordingly, for example, the discrimination layer can have a planar pattern substantially corresponding to planar regions of the separator 12 where the partitions 14 are disposed (hereinafter referred to as partition-corresponding regions). The discrimination layer does not have to have the color and/or image over the entire partition-corresponding regions. For example, the color and/or image may be formed in a frame pattern so as to surround the peripheries of the array regions 10, or may be formed partially near the peripheries of the array regions 10.

The discrimination layer provided for discrimination of the array regions 10 preferably has an appropriate color if the substrate 4 is a transparent substrate. For example, the array regions 10 can readily be recognized if any color is imparted to the partition-corresponding regions of the separator 12 or to part thereof. In addition, the individual array regions 10 can readily be discriminated and identified if the discrimination layer has images, such as numbers, letters, symbols, graphics, or combinations thereof, that allow identification of the individual array regions 10 near the peripheries of the array regions 10 in the partition-corresponding regions of the separator 12.

The discrimination layer may be formed of, for example, a colored film corresponding to the shape of the separator 12, or may also be formed of a print layer, or a film having a print layer, of the same shape. The print layer is preferred because any color or image can be imparted by printing. The discrimination layer does not have to be composed of a single layer and may also be composed of two or more layers stacked on top of each other. The two or more layers can have different colors or images.

The discrimination layer may be disposed in any site of the separator 12 as long as the array regions 10 can be discriminated. Specifically, the discrimination layer may be disposed on the top surface of the separator 12, although the discrimination layer is preferably disposed inside the top surface, that is, on the substrate 4 side. The discrimination layer can be disposed on the substrate 4 side to inhibit a colorant, such as ink, used to form the discrimination layer from reacting with the solutions under examination.

The discrimination layer can be used not only for discrimination and identification of the array regions 10, but also for discrimination of the probe array itself. Use of such a color or image for discrimination of the probe array reduces the likelihood of misoperation. In addition, any color or image can be imparted according to the manner and type of signal detection. Use of the discrimination layer improves the reliability of operation because the operation can be performed while checking the positions of the array regions 10. Furthermore, the discrimination layer facilitates discrimination of the two sides of the probe array. In addition, if the discrimination layer has a color or image with a high light-shielding effect, it can suppress deterioration of the underlying adhesive layer to suppress a decrease in the adhesion between the separator 12 and the substrate 4.

The discrimination layer does not have to be provided on the separator 12. For example, the discrimination layer may be separately provided on the surface of the substrate 4 after the formation of the probe-immobilizing layer 5 and before the attachment of the separator 12. Alternatively, the discrimination layer may be provided on the surface of the substrate 4 prior to the formation of the probe-immobilizing layer 5, or may be provided on the back surface of the substrate 4.

As shown in Fig. 2, the separator 12 is preferably attached to the surface of the probe-immobilizing layer 5 on the substrate 4. If the probe-immobilizing layer 5 is formed before the attachment of the separator 12, the probe-immobilizing layer 5 is uniformly formed on the surface of the substrate 4 without the effect of the presence of conventional hydrophobic regions for partitioning the array regions 10 or a treatment therefor. This allows a surface-finished layer, such as the probe-immobilizing layer 5, to be formed in the peripheral regions of the array regions 10 adjacent to the partitions of the separator 12 as uniformly as in the other portions. If the substrate 4 has partitions of a height corresponding to part of the height of the partitions 14, the separator 12 may be attached so as to be fitted to the partitions. If the substrate 4 constitutes part of the partitions 14, the entire surface of the substrate 4 where the array regions 10 are to be formed, including the regions where the partitions are to be formed, is preferably subjected to surface finishing to form the probe-immobilizing layer 5 before the attachment of the separator 12, as in the case of the flat substrate 4. The material of the separator 12 is not particularly limited, and examples include resins such as acrylic resins, thermoplastic elastomers, natural or synthetic rubbers, silicones, polyolefins, polyamides, polyimides, vinyl halides, and polycarbonates. The separator 12 is preferably flexible, and may also be transparent.

Preferably, the hydrophobic regions 18 are disposed on at least part of the separator 12. The hydrophobic regions 18 disposed on the separator 12 provide water repellency against aqueous solutions under examination to suppress contamination of the solutions under examination between the array regions 10. This also results in increased amounts of solutions retained within the array regions 10 exceeding the intrinsic capacity of the cavities 7. In the present invention, hydrophobic regions at least mean regions having water-repellent surface properties, preferably, regions having higher water repellency than general sodium silicate glass that is not subjected to, for example, hydrophilic treatment.

If the hydrophobic regions 18 are exposed in the cavities 7, they can produce, for example, a driving force acting to promote natural convection of the solutions under examination in the cavities 7. This results in, for example, excellent development of an interaction between substances, such as hybridization.

The hydrophobic regions 18 are preferably disposed on the tops or top surfaces of the partitions of the separator 12 in view of suppressing leakage and contamination of the solutions under examination and improving the ability of the cavities 7 to retain the solutions under examination. The tops or top surfaces herein refer to portions or surfaces of the partitions 14 facing away from the substrate 4. In view of promoting the substance interaction in the cavities 7, the hydrophobic regions 18 are preferably formed in regions of the separator 12 which are exposed in the cavities 7, for example, inside the partitions 14 (on the array region 10 side).

In general, the water repellency of the hydrophobic regions 18 can be represented as water contact angle on a flat surface. In the present invention, the water contact angle of the hydrophobic regions 18 is preferably 30° or more, more preferably 60° or more, still more preferably 70° or more, and most preferably 90° or more. The contact angle refers to the angle of contact between a droplet and a horizontal solid plate on which the droplet is placed. The contact angle used may be a static contact angle, an advancing or receding contact angle, represented as a critical value, or a dynamic contact angle, although a static contact angle measured by a drop method is preferably used.

The types of drop method used for measuring a static contact angle include (1) the tangent method, (2) the θ/2 method, and (3) the three-point clicking method. In the method (1), the tangent method, the contact angle of a droplet is directly determined using a reading microscope, for example, by moving a cursor to the tangent of the droplet. In the method (2), the θ/2 method, the contact angle of a droplet is determined by doubling the angle between a straight line connecting either side of the droplet and its apex and a solid surface. In the method (3), the three-point clicking method, the contact angle of a droplet is determined through image processing by clicking two contacts of the droplet with a solid surface and the apex of the droplet on, for example, a computer screen. Among these drop methods, the methods (2) and (3) are preferably used to determine the contact angle.

The hydrophobic regions 18 can be formed using a hydrophobic material as the material of the separator 12 itself or by providing a hydrophobic material and/or a hydrophobic (water-repellent) surface or layer in the regions where the hydrophobic regions 18 are to be formed. Examples of the hydrophobic material used for the hydrophobic regions 18 include polycarbonates, polyolefins such as polyethylene and polypropylene, vinyl halides, polyamides, polyimides, acrylic resins, and fluorides and chlorides of such resins. Examples of water-repellent surfaces include surfaces of various materials roughened by chemical modification or mechanical processing so as to have a contact angle of 60° or more.

The separator 12 is attached to the substrate 4 on which the probes 6 are immobilized, thus constituting the probe array 2. The separator 12 itself, before being attached to the substrate 4, independently constitutes an embodiment of the present invention. The separator 12 before attachment to the substrate 4 can have an adhesive layer 16 on the surface to be attached to the substrate 4. The adhesive layer 16 is preferably protected by a removable layer before being used.

The surface of the substrate 4 of the probe array 2 is uniformly finished, for example, the probe-immobilizing layer 5 is uniformly formed thereon, because the surface is not subjected to a conventionally employed treatment for forming hydrophobic regions and thus has no hydrophobic regions. This allows the probe-immobilizing layer 5 to be uniformly formed in the entire array regions 10, thus providing, for example, a uniform pattern of the minute regions 8 of the probes 6 in the array regions 10 and a uniform degree of probe immobilization. That is, the probe array 2 solves conventional problems such as deformation of the shapes of minute regions 8 of the probes 6 and variations in immobilization efficiency or low immobilization efficiency in the peripheral regions of the array regions 10.

Accordingly, the probe array 2 of the present invention provides a higher percentage (%) of effective probing area in the array regions 10 than conventional arrays. The percentage of effective probing area is the percentage of effective probing area (Se) to the design area (Sd) of the array regions (see the following equation (1)):

$$\text{Percentage (\%) of effective probing area} =$$

$$Se(mm^2)/Sd(mm^2) \times 100 \ldots (1)$$

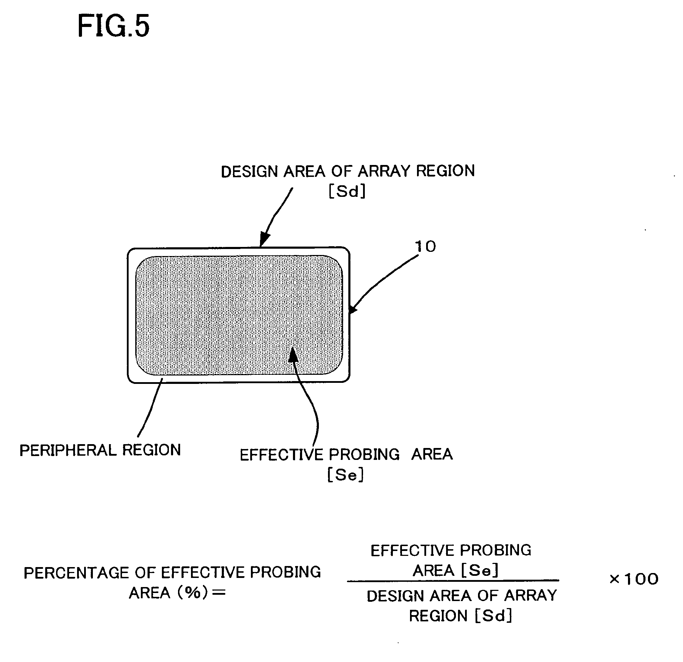

The design area (Sd) of the array regions is the area of each array region 10 surrounded by the partitions 14. The effective probing area (Se) is the area of a region where a probe-object interaction can be detected with a predetermined accuracy or more in each array region 10, specifically, the area of a region where the coefficient of variation of signal intensity based on the interaction is 20% or less. For hybridization using nucleic acid probes, for example, the effective probing area is the area of a region where the coefficient of variation of signal intensity, such as fluorescence intensity, based on the hybridization is 20% or less. Fig. 5 is a diagram illustrating the concept of the percentage of effective probing area.

The percentage of effective probing area depends on the design area (Sd) of the partitioned array regions 10. If Sd is 90 mm$^2$ or less, the percentage of effective probing area is preferably 70% or more. For conventional arrays, the percentage of effective probing area does not reach 70% at the above Sd (see Example 1). The percentage of effective probing area is preferably 80% or more, more preferably 85% or more, at the above Sd.

In the partitioned array regions 10 of the probe array 2 of the present invention, the coefficient of variation of signal intensity based on the interaction between the probes 6 and the samples under examination can be controlled to 20% or less in the regions other than those within the distance of 0.8 mm from the partitions 14. Conventional arrays cannot ensure such uniformity unless regions within the distance of about 1 mm from hydrophobic regions corresponding to the partitions 14 are excluded. Preferably, the coefficient of variation is controlled to 20% or less in the regions other than those within the distance of 0.6 mm, more preferably 0.4 mm, most preferably 0.3 mm, from the partitions 14. The coefficient of variation in such array regions is more preferably 15% or less, still more preferably 10% or less. These determinations can be made by measuring the signal intensity of the minute regions 8 arranged in lines separated from the partitions 14 by a predetermined distance and calculating the coefficient of variation.

(Method for producing probe array)

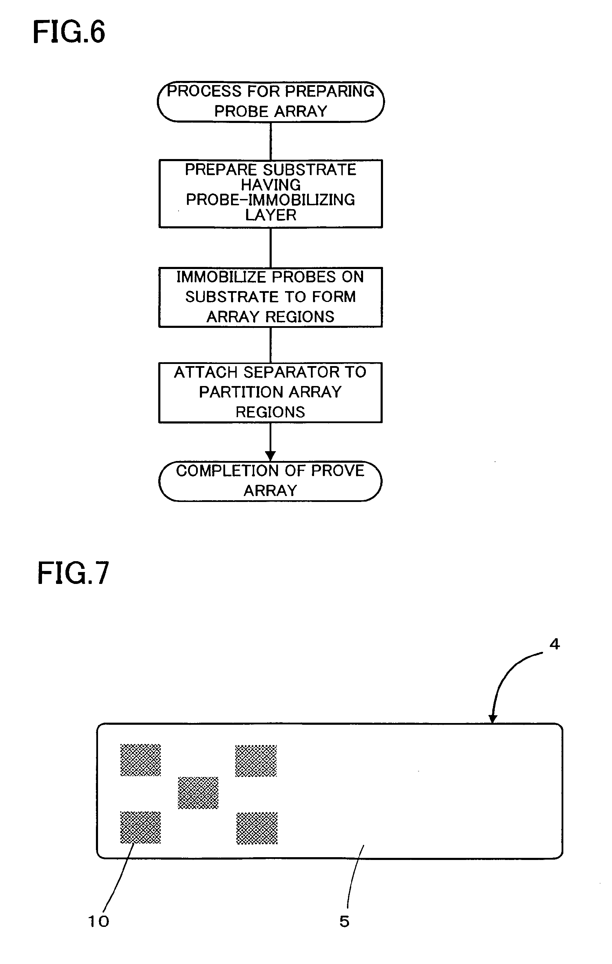

Next, a method of the present invention for producing a probe array will be described. The production method of the present invention will be described with reference to Fig. 6, using the probe array 2 shown in Figs. 1 and 2 as an example.

The method of the present invention for producing a probe array includes a step of immobilizing many probes 6 on the substrate 4 in a pattern where the partitioned array regions 10 can be formed and a step of partitioning the probes 6 into the array regions 10 of the pattern by attaching the separator 12 to the surface of the substrate 4 where the probes 6 are immobilized. The separator 12 includes the partitions 14 capable of partitioning the surface of the substrate 4 into a plurality of regions.

In the step of immobilizing the probes 6, the probes 6 are supplied onto the substrate 4, which has the probe-immobilizing layer 5, by any method. The probes 6 are preferably supplied so as to form the minute regions 8. The method for supplying the probes 6, for example, is not particularly limited, as described above, and methods of synthesizing the probes 6 on the surface of the substrate 4 are not excluded. The probes 6 are supplied in the pattern where the array regions 10 can be formed in the subsequent stage. Referring to Fig. 7, for example, a certain number of array regions 10 where many minute regions 8 are arranged are formed on the substrate 4. This pattern corresponds to the pattern of the array regions 10 partitioned by the partitions 14 of the separator 12 attached in the subsequent stage. The substrate 4 may have recesses corresponding to the array regions 10 or protrusions corresponding to at least part of the height of the partitions 14. In this case, preferably, the probe-immobilizing layer 5 is also provided on such portions.

The probes 6 are immobilized on the substrate 4, on which the pattern of the minute regions 8 has been formed, by treatments appropriate to the types of the probes 6 and the probe-immobilizing layer 5, for example, heat treatment, immersing in a liquid, and dehydration and cleaning.

In this method, no treatment for forming hydrophobic regions for partitioning is performed, and thus no hydrophobic regions are present on the substrate 4, prior to the immobilization of the probes 6. Accordingly, the probe-immobilizing layer 5 is uniformly provided over the substrate 4, so that the probes 6 can be immobilized on the probe-immobilizing layer 5 more reliably in better conditions.

Thus, a probe array can be provided which has many probes 6 immobilized on the substrate 4 in the pattern where the partitioned array regions 10 can be formed. When the probe array is used, the separator 12 is attached to partition the array regions 10. Accordingly, the probe array can be stored and distributed with the separator 12 detached and the array regions 10 unpartitioned.

The separator 12 is then attached to the surface of the substrate 4 on which the probes 6 have been immobilized. Because the outermost layer of the substrate 4 is the probe-immobilizing layer 5 on which the probes 6 are immobilized, the separator 12 is attached to the probe-immobilizing layer 5. The separator 12 has the partitions 14 capable of partitioning the surface of the substrate 4 into a plurality of regions. The array regions 10 can be partitioned by attaching the separator 12 to the surface of the substrate 4. The method and form of attachment of the separator 12 is not particularly limited. The separator 12 may be attached using the adhesive layer 16. Alternatively, the substrate 4 and the separator 12 may be held together using, for example, a jig.

The probe array 6 thus produced can have the structures described above. Accordingly, various forms of probe arrays 6 as described above can be produced by the method of the present invention.

(Method for hybridizing nucleic acid)

Nucleic acid probes can be used as the probes of the probe array of the present invention to provide a method for hybridizing nucleic acid in which nucleic acid samples contained in many or different solutions under examination can be simultaneously hybridized and assayed. Specifically, the method for hybridizing nucleic acid includes a step of hybridizing nucleic acid samples under examination with nucleic acid probes 6 by supplying solutions containing the nucleic acid samples under examination into the partitioned array regions 10 of the probe array 2. In this hybridization method, open cavities 7 for hybridization are preferably defined in the array regions 10. In the hybridization step, the solutions under examination are preferably supplied into the open cavities 7 to perform hybridization under humidified conditions. This allows many nucleic acid solutions under examination to be simultaneously assayed without being contaminated.

(Reaction method using probe array)

In a reaction method of the present invention using a probe array, the probe array includes one or more array regions, partitioned by partitions, where many probes are immobilized on a substrate. Preferably, a reaction is caused by supplying solutions under examination into the array regions with the partitions having a first height, and cleaning or detection of a reaction product after the reaction step is performed without the partitions present or with the height of the partitions reduced to a second height lower than the first height. For example, the first height is preferably 10 to 1,000 $\mu$m, and the second height is preferably less than 60 $\mu$m, more preferably 50 $\mu$m or less. Also, the second height is preferably 40 $\mu$m or more. Although the partitions 14 may be removed, the partitions 14 can effectively suppress contamination between the array regions 10 if the height of the partitions 14 is reduced by removing the tops of the partitions 14 so that the remaining partitions 14 have the above height or more.

In the reaction using the probe array having the partitioned array regions 10, the height of the partitions 14 may also be changed according to need by attaching a separator capable of defining the partitions 14 for each case, although the separator 12 of the present invention, whose height can be changed, is preferably used. The separator 12 allows the height of the partitions 14 to be readily changed according to the series of steps from reaction to detection. The separator 12 can therefore be used to supply sufficient amounts of solutions under examination while effectively suppressing contamination of the solutions under examination. In addition, the separator 12 facilitates cleaning, and can have a thickness appropriate to signal detection. If the separator 12 has overhanging portions, the height of the partitions 14 can be reduced because the overhanging portions can effectively suppress contamination of the solutions under examination.

EXAMPLE 1

Specific examples of the present invention will now be described. This example is intended to compare the uniformity of a surface-finish coat formed on a substrate after chemical operation (printing for imparting water repellency) and that of a surface-finish coat formed on a substrate before a separator sheet is laminated thereon. The comparison was performed by calculating the coefficient of variation (CV: (standard deviation/average) $\times$ 100 (%)) of fluorescence intensity measured in hybridization on three substrates for each line along the partitions, determining the distance at which the CV was stabilized, and evaluating coat uniformity based on the magnitude of the percentage of effective hybridization area. The percentage of effective hybridization area is defined as follows: (the area of the array regions where a stable CV was achieved/the design area of the array regions) $\times$ 100 (%).

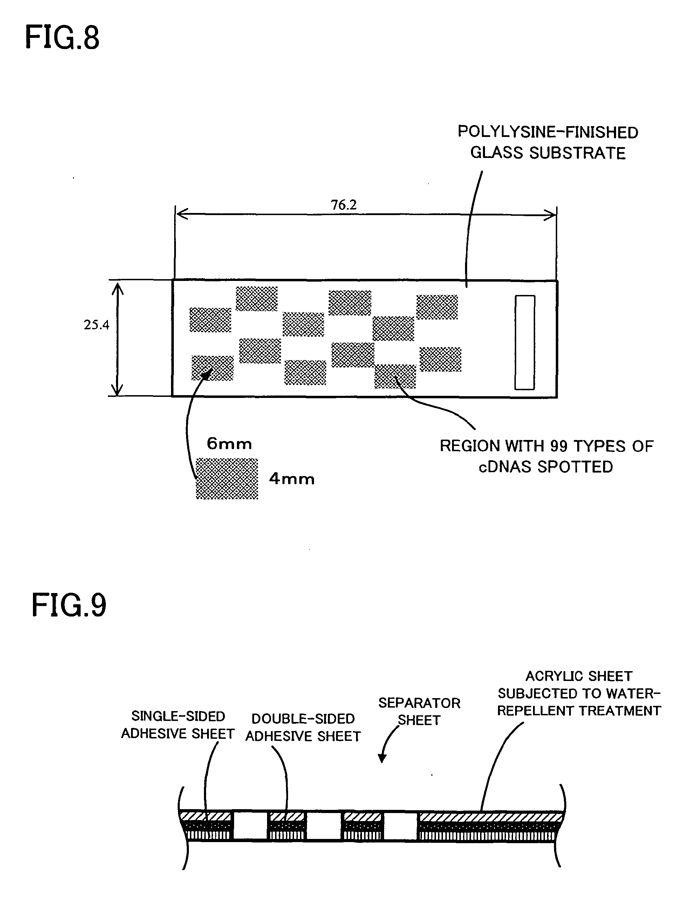

First, 99 types of rat-derived cDNAs were spotted in each of separate regions on glass substrates coated with poly-L-lysine, as shown in Fig. 8. These glass substrates were subjected to a heat treatment at 80°C for one hour, an immersing treatment in a blocking solution (for 15 minutes), an immersing treatment in boiling sterile water (for 3 minutes), dehydration with ethanol, and centrifugal drying to prepare nucleic acid probe arrays (unpartitioned). The blocking solution used was 70 mM succinic anhydride, 0.1 M sodium borate (pH 8.0), and 1-methyl-2-pyrrolidinone.

Separator sheets having a structure shown in Fig. 9 for partitioning the array regions were prepared as follows. First, acrylic sheets (t = 0.037 mm) were prepared, each having a surface subjected to a silicone-based water-repellent treatment (water contact angle: 110°). Laminates were then prepared by laminating double-sided adhesive sheets and single-sided adhesive sheets on the surfaces of the acrylic sheets opposite the water-repellent surfaces. These laminates were punched in a pattern corresponding to the separate regions shown in Fig. 8.

These separator sheets were laminated on the unpartitioned nucleic acid probe arrays to prepare nucleic acid probe arrays of this example.

Nucleic acid probe arrays of a comparative example were prepared as follows. First, the pattern shown in Fig. 8 was printed in advance on glass substrates using a hydrophobic material. The hydrophobic material was then solidified by heating before the substrates were coated with poly-L-lysine. These glass substrates were subjected to cDNA spotting, heating treatment, blocking, cleaning, and drying as described above to prepare nucleic acid probe arrays of the comparative example without laminating separator sheets.

Next, Cy3-labeled cDNAs were prepared using 1 $\mu$g of rat-derived mRNAs per substrate according to the procedure described in Cell Technology (Saibo Kogaku) Vol. 18, No. 7, 1999. These solutions were applied to the DNA arrays in an amount of 8 $\mu$l for each array region to perform hybridization at a final concentration of 5X SSC and 0.5% SDS. These probe arrays were placed in a tight box in which 3 ml of sterile water was sealed and were left at rest to perform hybridization. The hybridization was performed at 42°C and a humidity of 100% RH for 16 hours.

After the predetermined time elapsed, the probe arrays were cleaned by being shaken in three solutions (a (2 $\times$ SSC and 0.1% SDS) solution, a (1 $\times$ SSC) solution, and a (0.1 $\times$ SSC) solution) in the above order, each for five minutes. After the cleaning, the probe arrays were dried using a centrifuge (at 1,000 rpm for three minutes) and were measured for fluorescence using a scanner (Scan Array 4000, manufactured by Packard BioChip Technologies, LLC). The measured fluorescence intensities were digitized using numerical analysis software (Gene Pix Pro, manufactured by Axon Instruments). For each of the example and the comparative example, the CV of the three substrates was calculated using Excel to determine the distance of the spots from the wall surfaces at which the CV was stabilized and, based on the distance, to calculate the percentage of effective hybridization area.

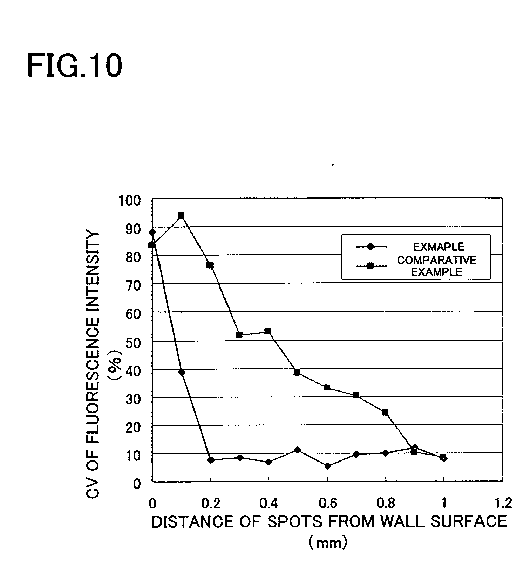

Fig. 10 shows that the CV of the probe arrays of the example (arrays to which water repellency was imparted by laminating the sheets after the surface coat was formed) was more stable at a short distance from the partitions than that of the probe arrays of the comparative example (arrays on which the surface coat was formed after water repellency was imparted). The distance from the partitions was 0.21 mm for the probe arrays of the example and was 0.88 mm for the probe arrays of the comparative example.

The percentages of effective hybridization areas based on the calculation results of the distance were as follows: Probe arrays of example: the distance from the partitions at which the CV was stabilized was 0.21 mm; and the percentage of effective hybridization area was [(4 - 0.21 $\times$ 2) $\times$ (6 - 0.21 $\times$ 2)]/24 = 83.2%.

Probe arrays of comparative example: the distance from the partitions at which the CV was stabilized was 0.88 mm; and the percentage of effective hybridization area was [(4 - 0.88 $\times$ 2) $\times$ (6 - 0.88 $\times$ 2)]/24 = 39.5%.

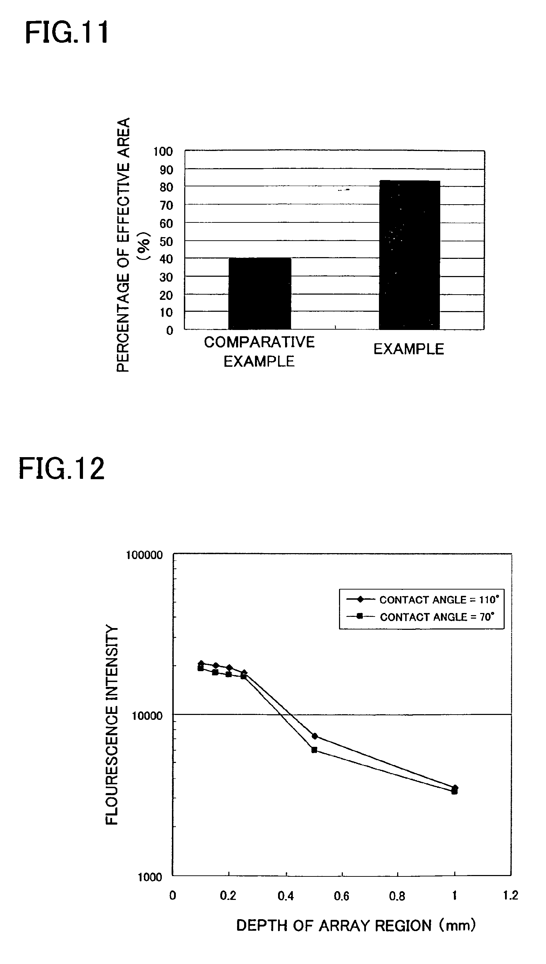

As also shown in Fig. 11, the percentage of effective hybridization area for the example was about 2.1 times that for the comparative example. These results demonstrate that the probe arrays of the present invention could suppress coat unevenness around the partitions to define highly uniform array regions, thus effectively immobilizing the nucleic acid probes.

EXAMPLE 2

This example is intended to examine the effect of the depth of the array regions on fluorescence intensity, a result of hybridization. Separator sheets varying in water repellency (water contact angles of 70° and 110°) and thickness were prepared as in Example 1 by changing the type of water repellent used for surface finishing and the thickness of the double-sided adhesive sheets and/or the single-sided adhesive sheets. Nucleic acid probe arrays of this example were prepared by the same operations as in Example 1 except that the above separator sheets were used. These probe arrays were subjected to hybridization to measure and digitize fluorescence intensity. The results are shown in Fig. 12.

Fig. 12 shows that the fluorescence intensity varied significantly around a depth of the array regions (thickness of the separator sheets) of 250 $\mu$m. These results demonstrate that the depth of the array regions is preferably 250 $\mu$m or less. There was no difference observed in fluorescence intensity due to the difference in water repellency (70° and 110° in this example).

EXAMPLE 3

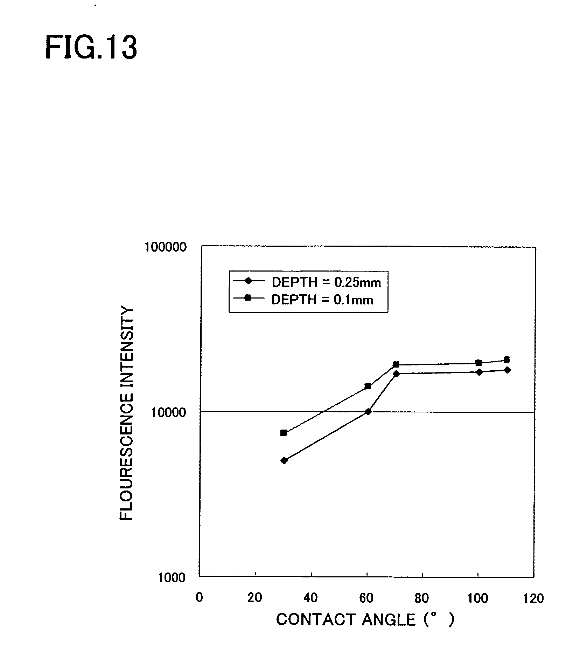

This example is intended to examine the effect of the water repellency of hydrophobic regions disposed on top surfaces of partitions of a separator sheet on fluorescence intensity, a result of hybridization. Separator sheets varying in water repellency (water contact angle) were prepared as in Example by changing, for example, the type of surface finishing on the acrylic sheets. Nucleic acid probe arrays of this example were prepared by the same operations as in Example 1 except that the separator sheets differing in water repellency were used. These probe arrays were subjected to hybridization to measure and digitize fluorescence intensity. The results are shown in Fig. 13.

Fig. 13 shows that the fluorescence intensity varied significantly around a water contact angle of 60° to 70°. These results demonstrate that the water repellency of the separator sheet is preferably 60° or more, more preferably 70° or more, in terms of water contact angle.

EXAMPLE 4

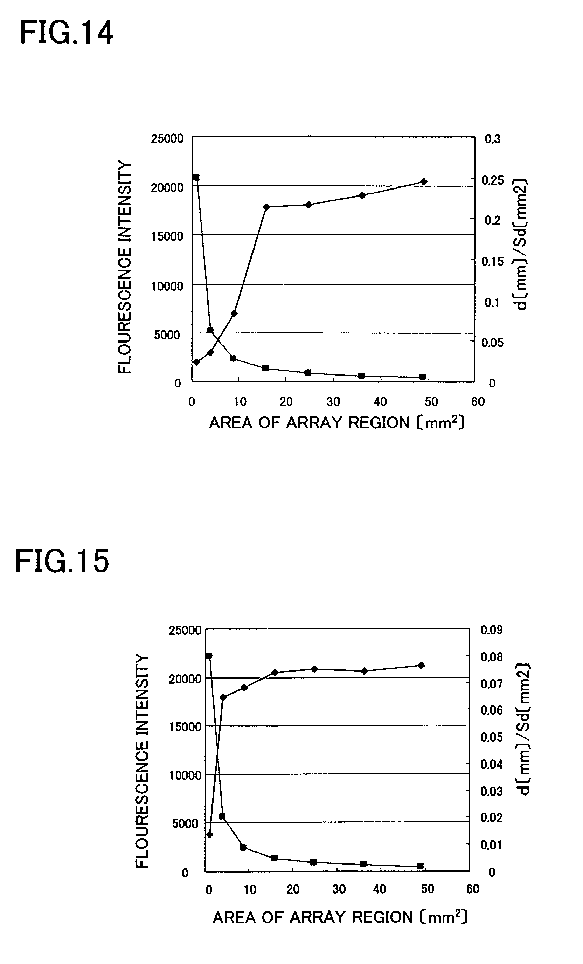

This example is intended to examine the effect of the design area of the array regions and the depth of the array regions on fluorescence intensity, a result of hybridization. Separator sheets with two different thicknesses (0.25 mm and 0.1 mm (depth of the array regions)) were prepared as in Example 1 by changing the thickness of the double-sided adhesive sheets and/or the single-sided adhesive sheets. These sheets were prepared using a total of seven types of dies with punch patterns corresponding to design areas of 1 mm$^2$, 4 mm$^2$, 9 mm$^2$, 16 mm$^2$, 25 mm$^2$, 36 mm$^2$, and 49 mm$^2$. The same surface finishing as in Example 1 was performed to impart water repellency. Probe arrays of this example were prepared by the same operations as in Example 1 except that the above separator sheets were used. These probe arrays were subjected to hybridization to measure and digitize fluorescence intensity. The results are shown in Figs. 14 and 15.

Fig. 14 shows the relationship between the design area of the array regions and the fluorescence intensity at a depth of the array regions (thickness of the separator sheets) of 0.25 mm. This graph shows that the fluorescence intensity varied significantly at a design area of the array regions of 15 mm$^2$ or more in comparison with design areas of less than 15 mm$^2$, and remained substantially constant. When the design area of the array regions was 15 mm$^2$, the ratio R of the depth (mm) of the array regions to the design area (mm$^2$) of the array regions was 0.02 (see the right vertical axis of Fig. 14). The ratio R was decreased with increasing design area of the array regions.

Fig. 15 shows the relationship between the design area of the array regions and the fluorescence intensity at a depth of the array regions of 0.1 mm. This graph shows that the fluorescence intensity varied significantly at a design area of the array regions of 5 mm$^2$ or more in comparison with design areas of less than 5 mm$^2$, and remained substantially constant. The ratio R at 5 mm$^2$ was 0.02 (see the right vertical axis of Fig. 15). The ratio R was decreased with increasing design area of the array regions.

The above results demonstrate that the ratio of the depth (mm) of the array regions to the design area (mm$^2$) of the array regions is preferably 0.02 or less.

EXAMPLE 5

In this example, a separator sheet having partitions whose height could be changed and one further having overhanging portions were prepared. In this example, the same operations as in Example 1, including the hybridization reaction and cleaning steps, were performed except that the separator sheets were prepared as follows.

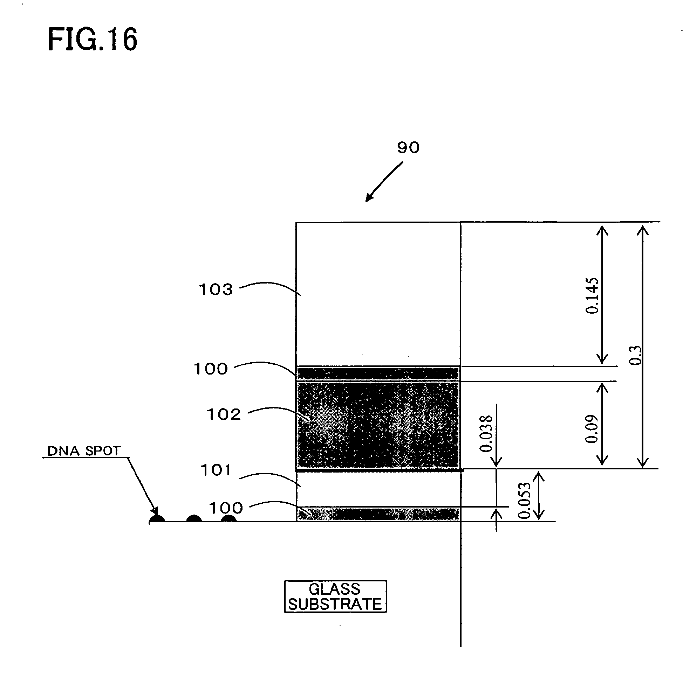

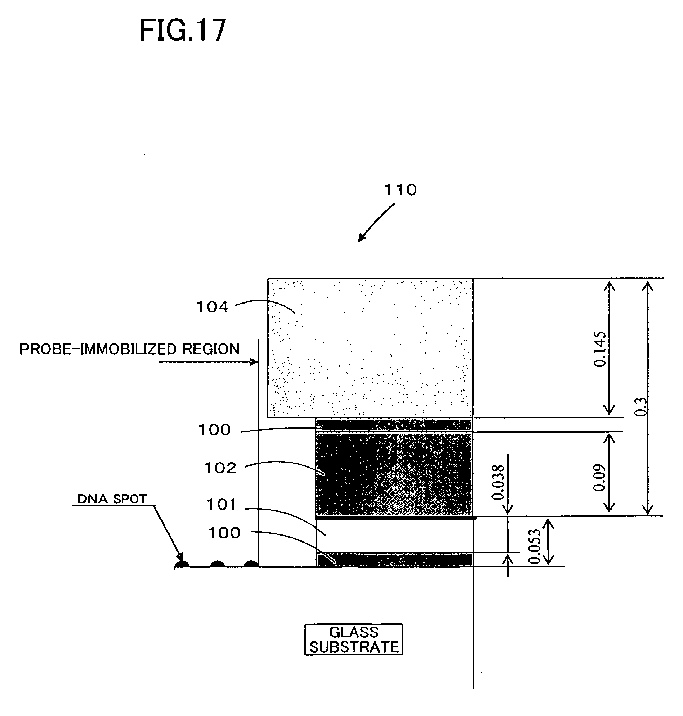

The separator sheets prepared in this example are shown in Figs. 16 and 17. A separator sheet 90 shown in Fig. 16 was prepared by laminating, from the substrate side, a polyethylene (PE) double-sided adhesive sheet 100 having acrylic double-sided adhesive layers on the top and bottom surfaces thereof, a 38 $\mu$m thick polyethylene terephthalate (PET) sheet 101 having a silicone-coated top surface, a 90 $\mu$m thick polyethylene sheet 102 having a silicone-based adhesive layer on the bottom surface thereof, another polyethylene double-sided adhesive sheet 101, and a 145 $\mu$m thick PET sheet 103, and then punching the laminate in the required pattern. The total thickness of the upper PE sheet 102, the double-sided adhesive sheet 101, and the PET sheet 103 was 300 $\mu$m. The interface between the PET sheet 101 and the PE sheet 102 was the most vulnerable interface of the entire sheet because the interface was formed of the silicone-based material.

A separator sheet 110 shown in Fig. 17 had the same structure as the separator sheet 90 shown in Fig. 16 except that the PET sheet 103 was replaced with a polycarbonate sheet 104 (thickness: 145 $\mu$m) overhanging in parallel toward the inside of the array regions. The separator sheet shown in Fig. 17 was prepared by separately punching the PE double-sided adhesive sheet 100, the PET sheet 101, the PE sheet 102, the PE double-sided adhesive sheet 100, and the PC sheet 104 before alignment and integration.

These separator sheets were laminated on glass substrates having array regions. Solutions under examination were supplied into cavities corresponding to the array regions in an amount of 8 $\mu$l for each array region. As a result, the separator sheets could avoid contamination of the solutions under examination when, for example, the arrays were stored in a sealed box for hybridization and the sealed box was placed in an oven. The substrate having the separator sheet shown in Fig. 17, which included the PC overhanging portions, could more stably retain the solutions in the cavities. For both separators having the separator sheets, the upper laminated portion with a thickness of 300 $\mu$m could readily be removed from the interface between the PET sheet 101 and the PE sheet 102 after the hybridization reaction. In addition, favorable reactivity could be achieved after the arrays were cleaned with the partition height reduced by removing the upper portion.

EXAMPLE 6

In this example, a separator sheet having a discrimination layer was prepared, and a probe array having the separator sheet was also prepared. In this example, the same operations as in Example 1 were performed, including the injection of the solutions under examination into the array regions.

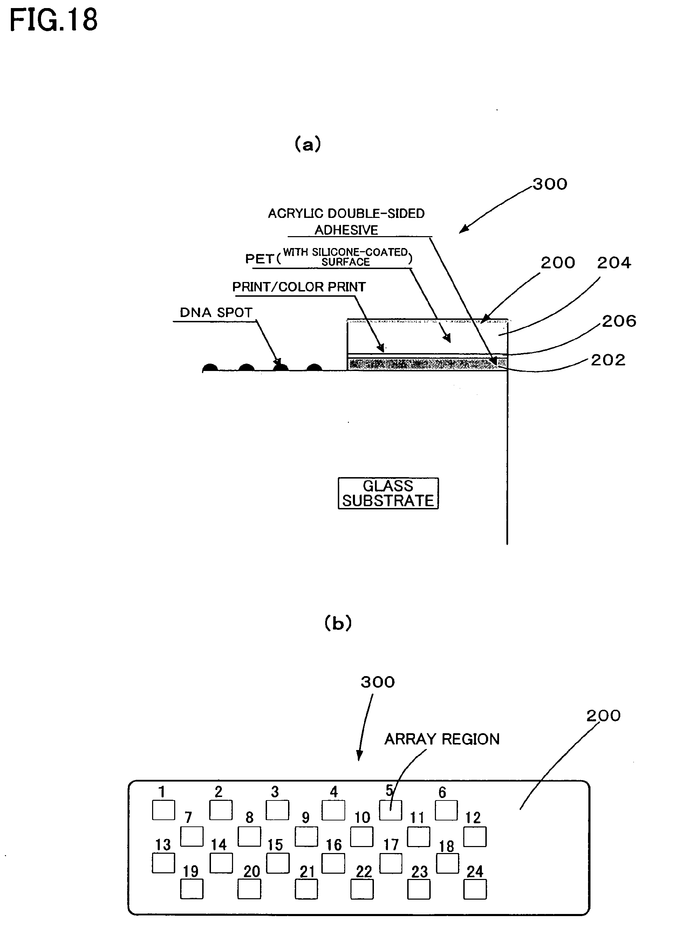

Fig. 18 shows a separator sheet 200 and probe array 300 prepared in this example. In Fig. 18(a), the separator sheet 200 was prepared by laminating, from the substrate side, a polyethylene (PE) double-sided adhesive sheet 202 having acrylic double-sided adhesive layers and a 38 μm thick polyethylene terephthalate (PET) sheet 204 having a silicone coating layer on the top surface thereof and a discrimination layer 206 on the bottom surface thereof, and then punching the laminate in the required pattern. White ink was applied to the entire discrimination layer 206 by printing, and the numbers 1 to 24 were printed at positions corresponding to the peripheries of the individual array regions on the visible side.

The separator sheet 200 was attached to a glass substrate on which probes had been spotted in 24 separate regions by the same operations as in Example 1 with the double-sided adhesive sheet 202 disposed therebetween to prepare the probe array 300, which had 24 array regions. In Fig. 18(b), the 24 transparent array regions were clearly shown in the white background of the probe array 300 and could readily be discriminated with reference to the numbers 1 to 24. The individual array regions could readily be recognized when the solutions under examination were injected into the array regions in an amount of 8 μl for each cavity, and thus the solutions under examination could be reliably injected into the array regions.

The present application claims the benefit of priority from Japanese Patent Application No.2005-089403 filed on March 25, 2005, Japanese Patent Application No.2005-315262 filed on October 28, 2005, International Application PCT/JP2006/301565 filed on January 31, 2006, and U.S. Provisional Patent Application No. 60/778,100 filed on March 2, 2006, the entire contents of all of which are incorporated herein by reference.

Industrial Applicability

The present invention can be used for manufacture of apparatuses for detecting nucleic acids, proteins, or synthetic or natural compounds in samples and industries involving the use of the detection results.

**Claims**

1. A probe array comprising:

   a substrate having one or more partitioned array regions where many probes are immobilized; and
   a separator attached to the substrate and including partitions partitioning the array regions.

2. The probe array according to Claim 1, wherein the substrate has a plurality of the array regions.

3. The probe array according to Claim 1 or 2, wherein the separator is attached to a surface of a probe-immobilizing layer on the substrate, the probes being immobilized on the probe-immobilizing layer.

4. The probe array according to Claim 3, wherein the separator is attached to the surface of the probe-immobilizing layer after the immobilization of the probes.

5. The probe array according to one of Claims 1 to 4, wherein the separator has a sheet shape and is fixed to the substrate with an adhesive layer disposed therebetween.

6. The probe array according to one of Claims 1 to 5, wherein the separator is detachably fixed to the substrate.

7. The probe array according to one of Claims 1 to 6, wherein hydrophobic regions are disposed on at least part of the separator.

8. The probe array according to Claim 7, wherein the hydrophobic regions are disposed on top surfaces of the partitions of the separator.

9. The probe array according to Claim 7 or 8, wherein the hydrophobic regions have a water contact angle of 60° or more.

10. The probe array according to one of Claims 7 to 9, wherein the hydrophobic regions contain a material selected from the group consisting of polycarbonates, polyolefins, polyamides, polyimides, acrylic resins, and fluorides and halides thereof.

11. The probe array according to one of Claims 1 to 10, wherein regions surrounded by the partitions of the separator partitioning the array regions constitute open cavities for interaction between the probes and samples under examination in the array regions.

12. The probe array according to one of Claims 1 to 11, wherein the design area of the array regions defined by the partitions is 0.3 to 2,000 $mm^2$.

13. The probe array according to one of Claims 1 to 12, wherein the substrate has 1 to 400 array regions.

14. The probe array according to one of Claims 1 to 13, wherein the design area (Sd) of the array regions defined by the partitions is 90 $mm^2$ or less, and the percentage of effective probing area represented by the following equation (1) is 70% or more:

$$\text{Percentage (\%) of effective probing area} =$$

$$Se(mm^2)/Sd(mm^2) \times 100 \ldots (1)$$

wherein Se is the area of a region where the coefficient of variation of signal intensity based on probe-sample interaction is 20% or less.

15. The probe array according to one of Claims 1 to 14, wherein the coefficient of variation of signal intensity measured by probing is 20% or less in regions other than those within the distance of 0.8 mm from the partitions in the partitioned array regions.

16. The probe array according to one of Claims 1 to 15, wherein the partitioned array regions have a depth (d) of 10 to 240 $\mu$m.

17. The probe array according to one of Claims 1 to 16, wherein the ratio (R) of the partitioned array regions which is represented by the following equation (2) is 0.02 or less:

$$R = d(mm)/Sd(mm^2) \ldots (2)$$

wherein d is the depth of the array regions and Sd is the design area of the array regions.

18. The probe array according to one of Claims 1 to 17, wherein the separator is configured so that the partitions can have different heights.

19. The probe array according to Claim 18, wherein the separator is configured so that the partitions can have different heights within the range of 10 to 1,000 $\mu$m.

20. The probe array according to one of Claims 1 to 19, wherein the separator is configured so that the height of the partitions can be reduced by removing at least part of the partitions.

21. The probe array according to one of Claims 18 to 20, wherein the partitions of the separator have a height of 60 $\mu$m or more when a reaction is caused by supplying samples under examination to the probes and have a height of less than 60 $\mu$m when a signal from a reaction product is detected after the reaction.

22. The probe array according to one of Claims 18 to 21, wherein the area of the array regions is 0.3 $mm^2$ or more.

23. The probe array according to one of Claims 18 to 22, wherein the separator has a laminated structure.

24. The probe array according to one of Claims 18 to 23, wherein the separator includes a vulnerable portion where the partitions can be at least partially removed.

25. The probe array according to Claim 24, wherein the separator is a laminate and the vulnerable portion is an interface between any layers of the laminate.

26. The probe array according to one of Claims 1 to 25, wherein the separator includes overhanging portions protruding from at least part of the tops of the partitions to above the array regions.

27. The probe array according to Claim 26, wherein the overhanging portions are disposed around the entire peripheries of the array regions.

28. The probe array according to Claim 26 or 27, wherein the overhanging portions include segments protruding from the partitions to above the array regions with hydrophobic regions disposed in at least regions of the segments where the segments can come in contact with solutions under examination.

29. The probe array according to one of Claims 26 to 28, wherein the height of lowermost ends of the overhanging portions is 40 to 990 $\mu$m.

30. The probe array according to one of Claims 26 to 29, wherein the overhanging portions protrude within such a distance as not to reach the probes immobilized in the array regions.

31. The probe array according to one of Claims 26 to 30, wherein regions defined by inner peripheral surfaces of the overhanging portions are regions where the probes are immobilized in the array regions.

32. The probe array according to one of Claims 26 to 31, wherein parts including the overhanging portions can be integrated with and/or removed from the rest of the partitions.

33. The probe array according to Claim 32, wherein the entire overhanging portions can be integrated with and/or removed from the rest of the partitions.

34. The probe array according to one of Claims 1 to 33, wherein the separator constitutes at least part of the height of the partitions partitioning the array regions.

35. The probe array according to one of Claims 1 to 34, wherein the substrate constitutes at least part of the height of the partitions partitioning the array regions.

36. The probe array according to one of Claims 1 to 35, further comprising a discrimination layer having a color and/or image that allows discrimination of the array regions.

37. The probe array according to Claim 36, wherein the discrimination layer is disposed on the substrate side of the separator.

38. The probe array according to Claim 36 or 37, wherein the separator includes the discrimination layer.

39. The probe array according to one of Claims 36 to 38, wherein the discrimination layer is formed by printing.

40. The probe array according to one of Claims 36 to 39, wherein the discrimination layer has a color that allows discrimination of the array regions.

41. The probe array according to one of Claims 36 to 40, wherein the discrimination layer has one or more marks selected from letters, numbers, symbols, and graphics to allow identification of the array regions.

42. The probe array according to one of Claims 1 to 41, wherein the probes are nucleic acid probes.

43. The probe array according to one of Claims 1 to 41, wherein the probes are protein probes.

44. A separator comprising partitions for partitioning one or more array regions where many probes are immobilized on a substrate.

**45.** The separator according to Claim 44, wherein the substrate has a plurality of the array regions.

**46.** The separator according to Claim 44 or 45, wherein hydrophobic regions are disposed on top surfaces of the partitions of the separator.

**47.** The separator according to one of Claims 44 to 46, wherein the separator is configured so that the partitions can have different heights.

**48.** The separator according to one of Claims 44 to 47, further comprising overhanging portions protruding from at least part of the tops of the partitions to above the array regions.

**49.** The separator according to one of Claims 44 to 48, further comprising a discrimination layer having a color and/or image that allows discrimination of the array regions.

**50.** The separator according to Claim 49, wherein the discrimination layer is disposed on the substrate side.

**51.** The separator according to Claim 49 or 50, wherein the discrimination layer is formed by printing.

**52.** The separator according to one of Claims 44 to 51, wherein the separator has a laminated structure.

**53.** The separator according to one of Claims 44 to 52, wherein the separator is used for the probe array according to one of Claims 1 to 43.

**54.** A probe array having one or more array regions where many probes are immobilized, the array regions being partitioned by the separator according to one of Claims 44 to 53.

**55.** The probe array according to Claim 54, wherein the probe array has a plurality of the array regions.

**56.** A method for producing a probe array, the method comprising:

a step of immobilizing many probes in one or more array regions on a substrate; and
a step of partitioning the many probes into the array regions by attaching a separator to a surface of the substrate where the probes are immobilized, the separator including partitions capable of partitioning the surface of the substrate.

**57.** The production method according to Claim 56, wherein a plurality of the array regions is formed.

**58.** A method for hybridizing nucleic acid, comprising a step of supplying solutions containing nucleic acid samples under examination into the array regions of the probe array according to Claim 42 to hybridize the samples under examination with the probes.

**59.** The hybridization method according to Claim 58, wherein
the probe array has open cavities for hybridization in the array regions; and
the hybridization step is performed under humidified conditions by supplying the solutions under examination into the open cavities.

**60.** A reaction method using a probe array, the method comprising a step of supplying solutions under examination into the array regions of the probe array according to Claim 43 to cause a reaction with the probes.

**61.** A reaction method using a probe array, the method comprising:

a reaction step of causing a reaction by supplying solutions under examination into one or more array regions, partitioned by partitions having a first height, where many probes are immobilized on a substrate of the probe array; and
a subsequent step of cleaning or detection of a reaction product after the reaction step, the subsequent step being performed without the partitions present or with the height of the partitions reduced to a second height lower than the first height.

62. The reaction method according to Claim 61, further comprising a step, prior to the reaction step, of forming the partitions having the first height on the substrate after the probes are immobilized on the substrate.

63. The reaction method according to Claim 61 or 62, wherein
the partitions having the first height in the reaction step include overhanging portions protruding from at least part of the tops of the partitions to above the array regions; and
the height of the partitions is reduced to the second height by removing the overhanging portions in the subsequent step.

64. The reaction method according to one of Claims 61 to 63, wherein the partitions are formed by attaching a separator capable of partitioning the array regions to a surface of the substrate.

65. The reaction method according to Claim 64, wherein the separator is a laminate.

# FIG.1

(a)

(b)

# FIG.2

FIG.3

FIG.4

# FIG.5

DESIGN AREA OF ARRAY REGION
[Sd]

10

PERIPHERAL REGION          EFFECTIVE PROBING AREA
[Se]

$$\text{PERCENTAGE OF EFFECTIVE PROBING AREA (\%)} = \frac{\text{EFFECTIVE PROBING AREA [Se]}}{\text{DESIGN AREA OF ARRAY REGION [Sd]}} \times 100$$

# FIG.6

```
┌─────────────────────────┐
│ PROCESS FOR PREPARING   │
│      PROBE ARRAY        │
└─────────────────────────┘
            │
┌─────────────────────────┐
│   PREPARE SUBSTRATE     │
│        HAVING           │
│  PROBE-IMMOBILIZING     │
│        LAYER            │
└─────────────────────────┘
            │
┌─────────────────────────┐
│  IMMOBILIZE PROBES ON   │
│   SUBSTRATE TO FORM     │
│     ARRAY REGIONS       │
└─────────────────────────┘
            │
┌─────────────────────────┐
│  ATTACH SEPARATOR TO    │
│    PARTITION ARRAY      │
│       REGIONS           │
└─────────────────────────┘
            │
            ▼
┌─────────────────────────┐
│  COMPLETION OF PROVE    │
│        ARRAY            │
└─────────────────────────┘
```

# FIG.7

# FIG.8

POLYLYSINE-FINISHED
GLASS SUBSTRATE

76.2

25.4

6mm

4mm

REGION WITH 99 TYPES OF
cDNAS SPOTTED

# FIG.9

ACRYLIC SHEET
SUBJECTED TO WATER-
REPELLENT TREATMENT

SEPARATOR
SHEET

SINGLE-SIDED      DOUBLE-SIDED
ADHESIVE SHEET  ADHESIVE SHEET

# FIG.10

# FIG.11

# FIG.12

FIG.13

## FIG.14

## FIG.15

# FIG.16

90

103

100

102

DNA SPOT

101

100

GLASS
SUBSTRATE

0.145

0.3

0.038

0.09

0.053

# FIG.17

110

104

PROBE-IMMOBILIZED REGION

100

102

DNA SPOT

101

100

0.145

0.3

0.038

0.09

0.053

GLASS
SUBSTRATE

# FIG.18

**(a)**

300

ACRYLIC DOUBLE-SIDED ADHESIVE

PET ( WITH SILICONE-COATED SURFACE )

PRINT/COLOR PRINT

DNA SPOT

200  204

206

202

GLASS SUBSTRATE

**(b)**

300

ARRAY REGION

200

| 1 | 2 | 3 | 4 | 5 | 6 |

7  8  9  10  11  12

13  14  15  16  17  18

19  20  21  22  23  24

# FIG.19

PROCESS FOR PREPARING
PROBE ARRAY

FORM HYDROPHOBIC
REGIONS FOR
PARTITIONING ARRAY
REGIONS ON SUBSTRATE

FORM
PROBE-IMMOBILIZING
LAYER

FORM PARTITIONED ARRAY
REGIONS WHERE PROBES
ARE IMMOBILIZED

COMPLETION OF PROBE
ARRAY

## INTERNATIONAL SEARCH REPORT

| | International application No. |
|---|---|
| | PCT/JP2006/306134 |

**A. CLASSIFICATION OF SUBJECT MATTER**
*G01N33/53*(2006.01), *C12M1/00*(2006.01), *C12N15/09*(2006.01), *G01N37/00* (2006.01)

According to International Patent Classification (IPC) or to both national classification and IPC

**B. FIELDS SEARCHED**

Minimum documentation searched (classification system followed by classification symbols)
*G01N33/53*(2006.01), *C12M1/00*(2006.01), *C12N15/09*(2006.01), *G01N37/00* (2006.01)

Documentation searched other than minimum documentation to the extent that such documents are included in the fields searched
Jitsuyo Shinan Koho          1922-1996   Jitsuyo Shinan Toroku Koho   1996-2006
Kokai Jitsuyo Shinan Koho    1971-2006   Toroku Jitsuyo Shinan Koho   1994-2006

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X/Y/A | JP 2002-357604 A  (Nisshinbo Industries, Inc.),<br>13 December, 2002 (13.12.02)<br>& EP 1251352 A        & CA 2382246 A<br>& US 2004/0161744 A | 1-13,34,35,<br>42-46,53-60/<br>36-41,49-51/<br>14-33,47,48,<br>52,61-65 |
| A | JP 2004-329136 A  (Aloka Co., Ltd.),<br>25 November, 2004 (25.11.04)<br>(Family: none) | 1-65 |
| A | JP 2004-515776 A  (Surface Logix, Inc.),<br>27 May, 2004 (27.05.04)<br>& WO 2002/048676 A      & AU 200243397 A<br>& US 2003/0032046 A      & EP 1339838 A | 1-65 |

| ☒ Further documents are listed in the continuation of Box C. | ☐ See patent family annex. |
|---|---|

\* Special categories of cited documents:
"A" document defining the general state of the art which is not considered   to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search<br>12 April, 2006 (12.04.06) | Date of mailing of the international search report<br>25 April, 2006 (25.04.06) |
|---|---|
| Name and mailing address of the ISA/<br>Japanese Patent Office | Authorized officer |
| Facsimile No. | Telephone No. |

Form PCT/ISA/210 (second sheet) (April 2005)

**INTERNATIONAL SEARCH REPORT**

International application No.

PCT/JP2006/306134

C (Continuation).    DOCUMENTS CONSIDERED TO BE RELEVANT

| Category* | Citation of document, with indication, where appropriate, of the relevant passages | Relevant to claim No. |
|---|---|---|
| A | JP 2004-226255 A  (Japan Science and Technology Agency),<br>12 August, 2004 (12.08.04)<br>(Family: none) | 1-65 |
| A | JP 2001-83158 A  (Fuji Photo Film Co., Ltd.),<br>30 March, 2001 (30.03.01)<br>(Family: none) | 1-65 |
| Y/A | JP 2004-28734 A  (Kabushiki Kaisha Makunika),<br>29 January, 2004 (29.01.04)<br>(Family: none) | 36-41,49-51/<br>1-35,42-48,<br>52-65 |

Form PCT/ISA/210 (continuation of second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002065274 A
- JP 2005089403 A
- JP 2005315262 A
- JP 2006301565 W
- US 77810006 P

**Non-patent literature cited in the description**

- *Cell Technology (Saibo Kogaku,* 1999, vol. 18 (7